# EUROPEAN PATENT APPLICATION

(11) **EP 3 747 347 A1**
(43) Date of publication of application: **09.12.2020**
(21) Application number: 20178543.3
(22) Date of filing: 05.06.2020
(51) Int. Cl.: A61B 1/015, A61B 1/303, A61B 1/32, A61B 1/018, A61B 1/04, A61B 1/31

(54) **A DEVICE FOR EXAMINING A BODY CAVITY**

(30) Priority: 06.06.2019 AU 2019100614
(71) Applicant: Pacific Endoscopic Imaging Pty Ltd, Newington, NSW 2127 (AU)
(72) Inventor: VAN DER WEEGEN, Clemens M, Newington, New South Wales 2127 (AU)
(74) Representative: Marles, Alan David

(57) **Abstract**

A portable device (500) for examining a body cavity, the device (500) adapted to allow air to be introduced into the body cavity, the device comprising: a tubular probe (1) having a first, forward end (4) and a second, rearward end (5); and a sealing element (506) disposed adjacent to the forward end (4) of the tubular probe (1), the sealing element (506) including a self-tightening sleeve portion (508) to engage the tubular probe (1), the sealing element (506) adapted to act as a seal for the body cavity to reduce egress of fluid therefrom by sealing against a circumferential zone of the internal tissue defining an orifice of the body cavity, the tubular probe (1) having a first lateral entry port (14) that allows for an elongate implement to be removably mounted in the device (500) and to be passed through the tubular probe (1) for introduction into the body cavity, such that the implement is manipulable by a user, the portable device (500) being connectable to an odour evacuation system having a filter (56) to remove the build-up of air in the body cavity, and wherein fluid being introduced into said body cavity via the tubular probe (1) is delivered via a conduit (51) from a pump (52), and a regulator (54) fluidly connected to the conduit (51) regulates the fluid to be within at least one predetermined range of pressure between at least an upper limit and a lower limit.

## Description

### TECHNICAL FIELD

This invention relates to examination devices of the kind used by physicians (such as gynecologists, doctors and nurses) to examine the interiors of body cavities and/or orifices thereof, and to facilitate sampling and/or surgical operations within the body cavities. In particular, the present invention is described with reference to an examination device for use in relation to the vagina, and which employs air regulation. However, it will be appreciated that the invention is not limited to use in relation to the vagina, and is also, for example, suitable for use in relation to other types of body cavities, such as the anus.

### BACKGROUND

There are a number of ways that physicians routinely examine the interior of the vagina of a patient. One such method is to employ a known dilating speculum comprising a tubular array of two or more rigid, elongate leaves or paddles, which are inserted, closed, into the vagina and expanded in a radially outward direction to open the vagina for inspection. This is often referred to as a "duck-bill" speculum.

Colposcopes are also routinely employed by gynecologists to examine the interior of the vagina through such a speculum, which provides an insertion path. Colposcopes typically comprise a binocular microscope and an illuminating unit which allows the physician to conduct the examination of the vagina, even though the colposcope is set up at about 300mm away from the vagina.

Some of the disadvantages of dilating speculums and colposcopes are discussed in the "Background Art" of US Patent No. 6,226,826 (Van Der Weegen), an earlier patent by the present inventor, summarised below.

US Patent No. 6,226,826 discloses an examination device for examining the interior of a body cavity, which is simple to use for close up examination and through which biopsy samples may be obtained and surgery conducted. Fig. 1 of US Patent No. 6,226,826 depicts an embodiment which is particularly suited to the examination of a vagina. The examination employs a tubular probe mounted onto a hand piece, the tubular probe having a shield at its fore end that is adapted to engage the tissue externally of the vaginal opening. The rear end of the tubular probe extends from the rear of the hand piece and is adapted to have a closure, with an eyepiece, fitted thereto. The eyepiece is able to have a camera fitted thereto. The handpiece contains a light source that is able to illuminate the tubular probe when it is inserted into the vagina to assist the examination thereof. The closure also has a sealable entry port for the insertion of a surgical implement or sampling device through the rear of the tubular probe. A problem associated with the location of the entry port, is that a physician finds it difficult to hold the hand piece in one hand with the shield pressed firmly against the external tissue of the vagina, whilst endeavouring to manipulate the surgical implement or sampler from the rear of the device whilst also trying to view inside the vagina. Another problem with this device is that the shield does not always provide an effective seal as it seals only on the external tissue, and requires air to inflate the sealed vagina in order for the cervix to be examined.

US 6,719,687 (Van Der Weegen), another earlier patent by the present inventor, discloses a vaginal speculum (or examination device) which attempted to overcome the sealing problems of US 6,626,826 by employing an annular sealing element in the form of a thick walled, domed shell of polyethylene foam fitted to the disposable tubular probe that is inserted into the vagina. The disposable tubular probe includes a domed sealing element that seals against a circumferential zone of the internal tissue defining the vaginal orifice, and is suitable for use with women having different sized and shaped vaginal orifices. The sealing element provides a low-pressure seal over a limited range of depths of penetration of the probe into the vagina and over a range of angular alignments of the probe. This prior art speculum relies upon the natural resilience of the so-called PC muscle surrounding the vaginal orifice to permit the orifice to expand and contract so as to allow penetration of the sealing element into the orifice and the maintenance of a seal therewith, throughout a limited range of depths of penetration. The sealing element of this prior art speculum can only form a low-pressure seal, and if the vaginal barrel is over pressurized, the zone where the sealing element contacts the vagina expands to allow the pressurising fluid, typically air, to escape.

With this type of device, the tubular probe is held by a hand piece for manipulation of the probe, and an eye piece enables an operator to aim the probe and directly view the cervical area of the vagina. An enclosure containing a light source and air pump is connected to the hand piece by a flexible tube. An optical fibre cable extending through the tube enables the cervical area of the vagina to be illuminated by light travelling along that cable and through the transparent walls of the probe. Pressurised air from the pump also flows along the flexible tube into the interior of the hand piece and then into the bore of the probe. The air supply rate can be regulated by the user of the speculum. The eyepiece also seals the trailing end of the probe to prevent leakage of air from that end. In this speculum the air regulation is simply a number of holes in the fluid delivery supply or handle that could be covered or uncovered by the user to alter the air being delivered. A problem with this speculum, as can be seen in Fig. 2 of US 6,719,687, was that the only way to introduce a surgical implement or other implement is through the closure/eyepiece fitted to the rear of the tubular probe. As such, this device suffers from the same manipulation problems that occur with the abovementioned device of US 6,626,826.

WO2009/059355 (Van Der Weegen), a further earlier patent publication by the present inventor, discloses a speculum (or examination device) having a lateral port that allows for an elongate implement, such as a sampling device (or surgical tool) to be removably mounted therein and passing through the speculum's tubular probe for introduction into the vagina, such that said implement may be manipulated by a user. A V-shaped guide member near the fore of the probe is offset from the central axis of the probe. This offset guide member ensures that there is not a large impact on the physician's field of vision. This speculum with its improved field of vision and with an illumination assembly and a digital camera in the handpiece, can be used with an associated computer system having a monitor. This allows the physician to examine the patient by viewing the image of the vaginal interior and cervix shown on the monitor, and may capture images thereof for storage on the computer or another associated storage device.

Like the arrangement of US 6,719,687 (Van Der Weegen), the speculum in WO2009/059355 is found by patients to be more comfortable than the prior art "duck-bill" speculums having the leaves and the like. However, this arrangement does suffer from a number of disadvantages. One such disadvantage is that in the event where the vagina is over pressurised and air escapes the seal zone, the patient generally becomes embarrassed as the noise generated as the air escapes the body cavity sounds similar to flatulence or to noises generated during some forms of sexual intercourse.

This embarrassment may deter some women from undergoing regular pap smear tests and cervical examinations. Secondly, because the speculum is now being used with a digital camera for image viewing and capture of images for later review, it is important to ensure that the vaginal barrel stays sufficiently pressurised during the image viewing and capture, but without the over-pressurising. As such, the crude air regulation of US 6,719,687 (Van Der Weegen) is not suitable.

### OBJECT OF THE INVENTION

It is an object of the present invention to substantially overcome or at least ameliorate one or more of the disadvantages of the prior art, or provide a useful alternative.

### SUMMARY OF THE INVENTION

According to one aspect, the present invention provides a portable device for examining a body cavity, the device adapted to allow air to be introduced into the body cavity, the device comprising:
a tubular probe having a first, forward end and a second, rearward end; and
a sealing element disposed adjacent to the forward end of the tubular probe, the sealing element including a self-tightening sleeve portion to engage the tubular probe, the sealing element adapted to act as a seal for the body cavity to reduce egress of fluid therefrom by sealing against a circumferential zone of the internal tissue defining an orifice of the body cavity,
the tubular probe having a first lateral entry port that allows for an elongate implement to be removably mounted in the device and to be passed through the tubular probe for introduction into the body cavity, such that the implement is manipulable by a user,
the portable device being connectable to an odour evacuation system having a filter to remove the build-up of air in the body cavity, and
wherein fluid being introduced into said body cavity via the tubular probe is delivered via a conduit from a pump, and a regulator fluidly connected to the conduit regulates the fluid to be within at least one predetermined range of pressure between at least an upper limit and a lower limit.

The pressure gauge is preferably in fluid communication with the conduit for setting the predetermined range of pressures.

The upper limit is preferably just below where over-pressuring of air occurs in the vagina, and the lower limit is preferably just above a pressure needed to keep the body cavity inflated for viewing.

The tubular probe preferably includes a guide means disposed adjacent the second, rearward end of the prove, the guide means adapted to guide the implement within the tubular probe, and wherein the guide means is a substantially V-shaped guide member.

The portable device preferably further includes an imaging device having an associated light source for emitting green, white, or ultraviolet blue light onto the body cavity.

According to another aspect, the present invention provides a device for examining a vagina, the device adapted to be mounted to a handpiece and for allowing air to be introduced into the vagina, the device comprising:
a tubular probe having a first, forward end and a second, rearward end; and
a sealing element disposed adjacent to the forward end of the tubular probe, the sealing element adapted to act as a seal for the vagina to reduce egress of fluid therefrom by sealing against a circumferential zone of the internal tissue defining a vaginal orifice,
the tubular probe having a first lateral entry port that allows for an elongate implement to be removably mounted in said device and passed through the tubular probe for introduction into the vagina, such that said implement is manipulable by a user,
wherein fluid being introduced into said vagina via the tubular probe is delivered via a conduit from a pump, and a regulator fluidly connected to the conduit regulates the fluid to be within at least one predetermined range of pressure between at least an upper limit and a lower limit.

Preferably, a pressure gauge is in fluid communication with the conduit.

Preferably, the pressure gauge is integrally housed with the regulator.

In one embodiment, the regulator is preferably a turn knob regulator.

Preferably, the upper pressure limit is just below where over-pressuring of air occurs in said vagina, and the lower pressure limit is just above the pressure needed to keep said vagina inflated for viewing.

Preferably, the fluid is air.

In another embodiment, the regulator is preferably an automated regulator including a pressure sensor and electronic control unit, and said regulator automatically regulates said air pressure within said predetermined range.

Preferably, the at least one predetermined range is a plurality of predetermined ranges that is selectively usable.

Preferably, the regulator includes an alarm operably connected to the electronic unit that is activatable outside the predetermined range.

Preferably, the device further includes a filter to remove the build-up of air in the vagina.

Preferably, the device further includes a fluid entry port associated with the tubular probe to allow for the introduction of fluid into the vagina.

Preferably, the fluid is acetic acid.

Preferably, the tubular probe includes a guide means disposed adjacent the second, rearward end, the guide means adapted to guide the implement within the tubular probe.

Preferably, the guide means is a substantially V-shaped guide member.

Alternatively, the guide means is a substantially keyhole-shaped guide member.

Alternatively, the guide means is a substantially ring-shaped guide member.

### BRIEF DESCRIPTION OF THE DRAWINGS

Preferred embodiments of the present invention will now be described, by way of example only, with reference to the accompanying drawings, in which:
Fig. 1 is a perspective view of a first embodiment of an examination device, with a sampling implement fitted thereto;
Fig. 2 is an exploded perspective view of the examination device shown in Fig 1;
Fig. 3a is an enlarged elevation view of the tubular probe component of the examination device shown in Fig. 1;
Fig. 3b is a plan view of the tubular probe shown in Fig 3a;
Fig. 3c is a bottom view of the tubular probe shown in Fig 3a;
Fig. 4 is an aft end view of the tubular probe shown in Fig 3a;
Fig. 5 is a cross-sectional schematic view of the tubular probe through lines V-V of Fig 4;
Fig. 6 is a transparent view of the fore end of the tubular probe of the examination device shown in Fig. 1, depicting the internal guide member;
Fig. 7 is an aft end view of a second embodiment of the tubular probe shown in Fig. 3a;
Fig. 8 is a cross-sectional schematic view of the second embodiment of the tubular probe through lines V-V of Fig. 7;
Fig. 9 is a transparent view of the fore end of the second embodiment of the tubular probe shown in Fig. 7, depicting a variation in the internal guide member;
Fig. 10 is an aft end view of a third embodiment of the tubular probe shown in Fig. 3a;
Fig. 11 is a cross-sectional schematic view of the third embodiment of the tubular probe through lines V-V of Fig. 10;
Fig. 12 is a transparent view of the fore end of the third embodiment of the tubular probe shown in Fig. 10, depicting a variation in the internal guide member;
Fig. 13 is a schematic of a first embodiment of a system incorporating the examination device of the present invention;
Figs. 14A and 14B are perspective views of the system shown in Fig. 13;
Fig. 14C is a side view of the system shown in Fig. 13;
Fig. 15 is a side schematic view of components of the system shown in Fig. 13;
Fig. 16 is a side schematic view of components of a second embodiment of a system incorporating the examination device of the present invention;
Fig. 17 is a side cross-sectional view of components of the system shown in Fig. 16;
Fig. 18 is a schematic of the second embodiment of the system shown in Fig. 16;
Fig. 19A is a side view of a tubular probe extension of the examination device of the present invention, being a gynecologist's speculum;
Fig. 19B is a perspective view of the tubular probe extension shown in Fig. 19A;
Fig. 20 is a front view of an illumination assembly connected to a camera on the examination device shown in Fig. 1;
Fig. 21 is a perspective view of a further embodiment of an examination device;
Fig. 22 is a perspective view of a sealing element of the examination device of Fig. 21;
Fig. 23 is a front view of the sealing element of Fig. 22;
Fig. 24 is a top view of another embodiment of the tubular probe; and
Fig. 25 is a cross-sectional schematic view along A-A of the tubular probe of Fig. 24.

### DETAILED DESCRIPTION OF THE EMBODIMENTS

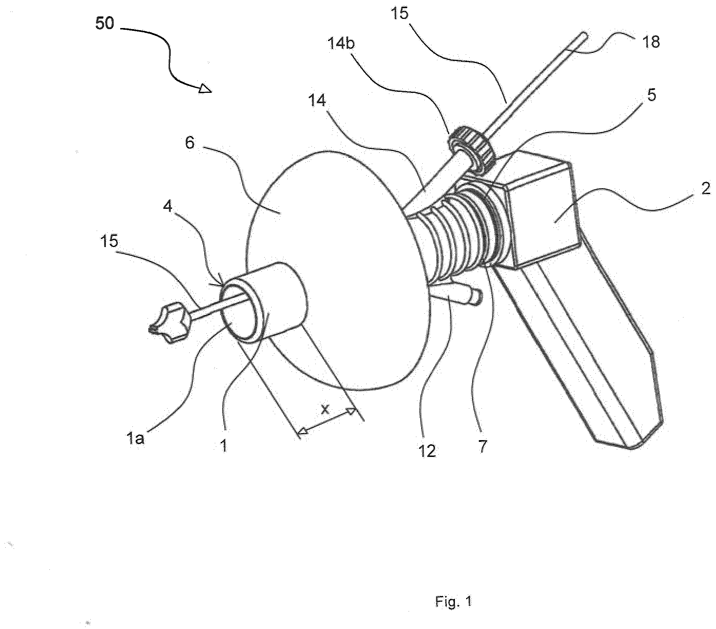

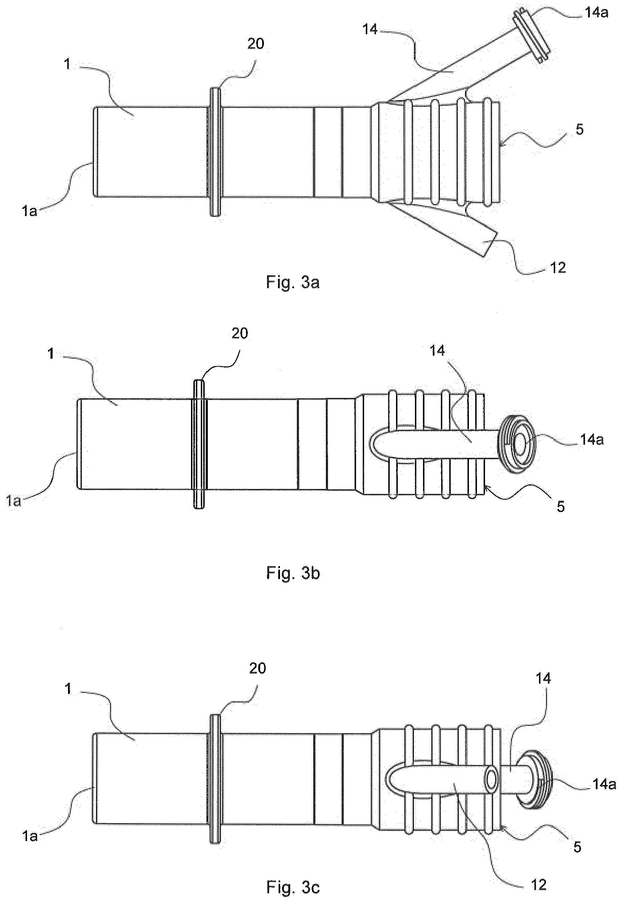

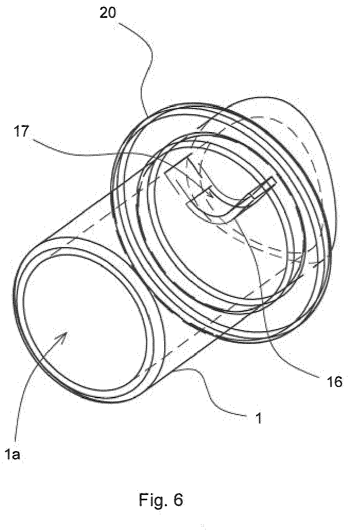

Figs. 1 to 6 depict an embodiment of an examination device (or doctor's speculum) 50 intended for use in the examination of a vagina or other body cavity. The examination device 50 is similar to that shown in WO2009/059355 (Van Der Weegen). The examination device 50 comprises an elongate tubular probe 1 adapted to be used in conjunction with a handpiece 2. The tubular probe 1 is preferably a colourless transparent plastic moulding which may, for example, be formed from acrylic, having a bore 1a. For ease of reference, tubular probe 1 is described as having a first (forward) end 4 and a second (rearward) end 5, with a longitudinal axis L1 extending therebetween. The examination device 50 includes a sealing element 6 disposed on the tubular probe 1. The sealing element 6 has a larger diameter than the body of the tubular probe 1.

The sealing element 6 is similar to "sealing element 13" depicted in Fig. 1 of US Patent No. 6719687 and functions in a similar fashion. The sealing element 6 is preferably formed from a disposable material, for example, a thick walled hemisphere (dome-shaped shell) formed from a closed cell, dense polyethylene foam. The sealing element 6 includes a hole at its centre through which the tubular probe 1 extends. It is envisaged that the tubular probe 1 and the hole of the sealing element 6 has a tight fit. A circular rib 20 is disposed on the tubular probe 1 to limit the rearward travel of the sealing element 6 relative to the tubular probe 1.

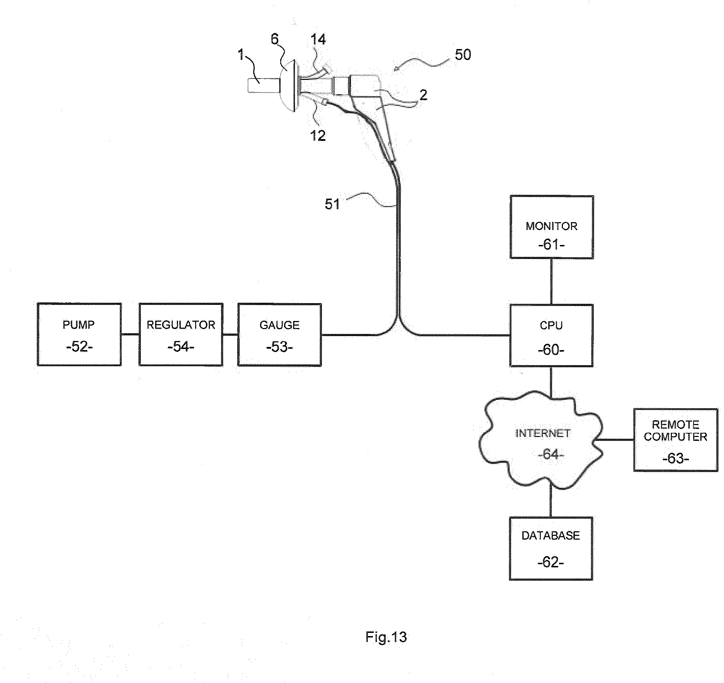

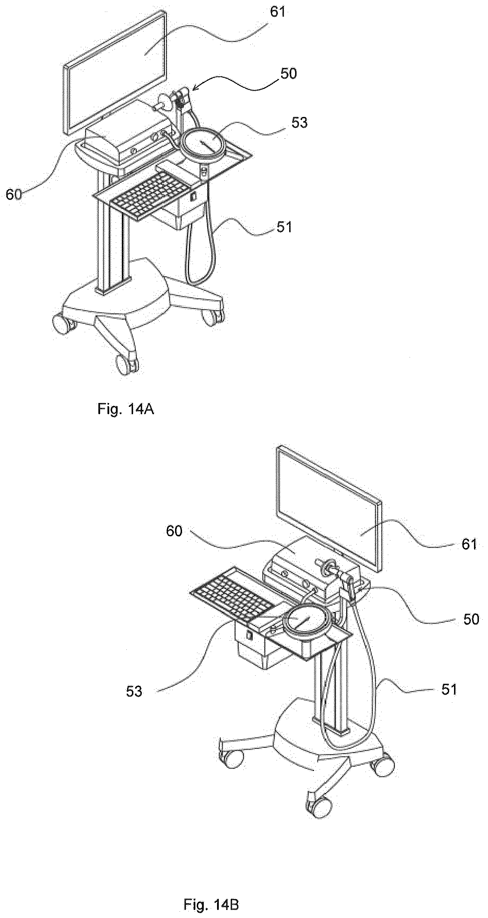

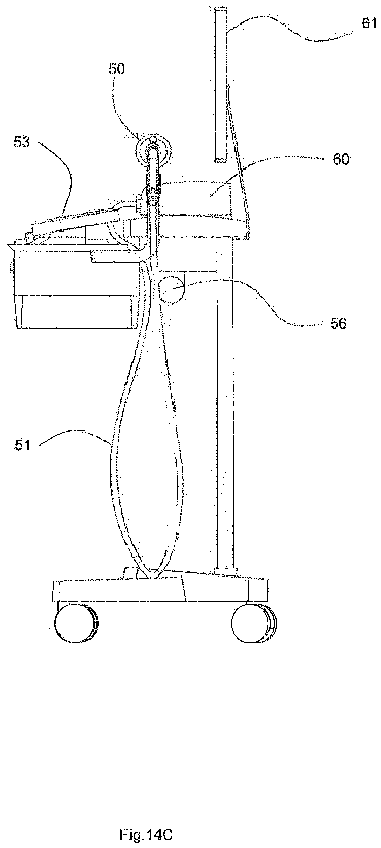

The handpiece 2 has a detachable mounting spigot 7 for mounting the tubular probe 1. The handpiece 2 also has an illumination assembly (not shown) and a digital imaging acquisition means (camera) for viewing and/or capturing an image (not shown), therein. As will be described in further detail below, the handpiece 2 is also adapted to be connected to a computer 60 or the like and transformer (not shown), from which a low voltage power source of approximately 3 to 12 volts to power the illumination assembly and the digital imaging acquisition means, is provided. As best shown in Figs. 13, 14a, 14b, and 14c, the computer 60 is connected to a monitor 61 to provide a graphical interface for the user.

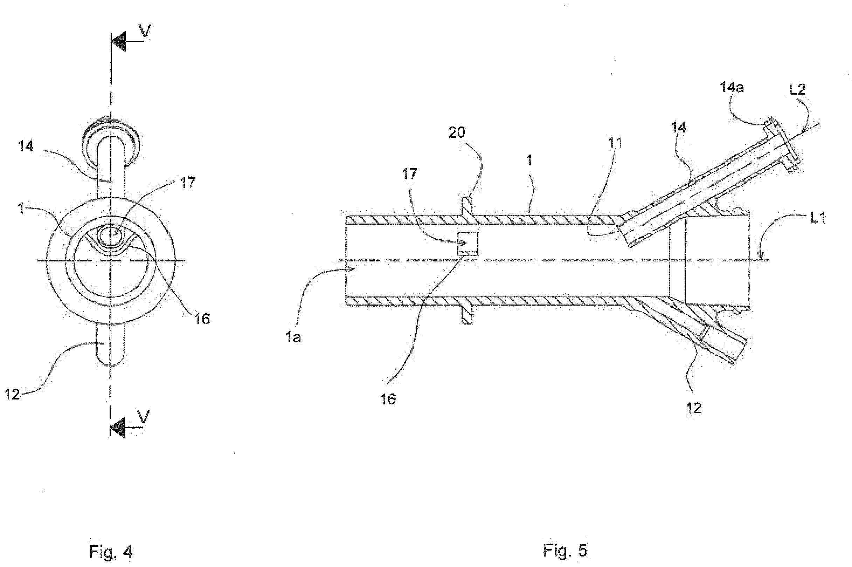

As best shown in Fig. 5, the tubular probe 1 comprises two ports, the first being a lateral entry port 11 for an implement 15 (such as a cervical brush, biopsy tool or the like) to pass through and extend into the bore 1a of the tubular probe 1, and the second being a fluid entry port 12 for introducing a fluid such as air into the examination device 50, for example to inflate a vaginal barrel/cavity of a patient. For ease of reference, the supply of fluid is not shown in the Figures.

A tubular guide member 14 projects externally from the lateral entry port 11 in a substantially rearward direction (i.e. towards the second (rearward) end 5 of the tubular probe 1). The tubular guide member 14 has a longitudinal axis L2 that is at an acute angle, preferably less than 45°, to the longitudinal axis L1 of the tubular probe 1. This arrangement allows for an elongate implement, such as a sampling implement 15 with a flexible plastic stem or the like (cervical brush), to be inserted through the first (forward) end 4 of tubular probe 1 and bent such that a rear end 18 of the implement 15 passes through the lateral entry port 11 and the tubular guide member 14, thereby guiding the rear end 18 of implement 15 away from the tubular probe 1 (as best shown in Fig. 1). A thread 14a is disposed on the tubular guide member 14 for receiving a grommet and seal arrangement 14b for engagement therewith. The stem of the implement 15 passes through the grommet and seal arrangement 14b as the implement 15 passes through the tubular guide member 14.

In order to keep the stem of the implement 15 centralised in the bore 1a of the tubular probe 1, a guide means may be located at or adjacent the second (rearward) end 4 of the tubular probe 1. It should, however, be understood that other shapes could be utilised for different types of applications or procedures. The guide means may, for instance, be a substantially V-shaped guide member 16 projecting from an inner surface or wall of the bore 1a of the tubular probe 1, as best shown in Figs. 4 and 5. The stem of the implement 15 passes through an aperture 17 disposed between guide member 16 and the inner surface or wall of the bore 1a of the tubular probe 1. It should be appreciated that, the location where the guide member 16 supports the stem of the implement 15 is offset from the longitudinal (i.e. central) axis L1 of the tubular probe 1. This may at least ensure that the guide member 16 and the location at which the stem of the implement 15 is supported by the guide member 16 does not obstruct the longitudinal (i.e. central) axis L1 of the tubular probe 1.

It should be understood that whilst this embodiment is described with reference to a sampling implement 15, it should be understood that the implement 15, may, in a different embodiment, be a surgical implement or other medical instrument or the like.

The examination device 50 is typically supplied to a user as separate unassembled components, i.e. the tubular probe 1, the handpiece 2 and the mounting spigot 7. In one example, the examination device 50 may be assembled for use by attaching the tubular probe 1 to handpiece 2 via the mounting spigot 7. Alternatively, the tubular probe 1 may be directly mounted to the handpiece 2 without the use of the mounting spigot 7. The separation of components at least allows the flexibility of use, depending upon the type of patient, and also ensures that the components may be disposed of and replaced for hygiene reasons. The various components of the examination device 50 may be connected by way of threaded engagement, snap fitting, or other forms of corresponding mechanical features.

The operation of the examination device 50 will now be described.

Once the components of the examination device 50 have been assembled, the sealing element 6 of the tubular probe 1 is located at a front (i.e. distal) end of the examination device 50. In one example, a physician wishing to examine a patient's vagina may bring the first (forward) end 4 of the tubular probe 1 towards the opening of the vagina and insert the tubular probe 1 in a like manner to that shown in Fig. 5 of US Patent No. 6719687, thereby effecting a seal between the sealing element 6 and the annular zone of the normally internal surface of the vaginal orifice (or the vaginal cavity/barrel). Then, in a similar fashion to that described in US Patent No. 6719687, the vaginal orifice or cavity/barrel may be inflated by air (or another fluid, for example CO₂ or the like) introduced into the examination device 50 via the fluid entry port 12. Referring to Fig. 1, for example, the physician may turn on the air supply when the first (forward) end 4 of the tubular probe 1 of the examination device or speculum 50 is placed at the entrance of the vagina. As the air from the air supply inflates the vagina, the vaginal entrance begins to dilate (i.e. open up). It will be appreciated that little to no force is required by the physician, thus making it easier for the tubular probe 1 to be inserted and for a proper seal to be effected by the sealing element 6. At the same time, the cervix, which faces the floor of the vagina, lifts up to the centre of the vagina once it is fully inflated, providing a better view for the user.

It is envisaged that the illumination assembly within the handpiece 2 projects light into the vaginal barrel. As the handpiece 2 is connected to the computer 60 and the monitor 61, and the digital imaging acquisition means (camera) for viewing and/or capturing an image is incorporated within the handpiece 2, the physician may examine the patient by viewing an image of the vaginal interior and cervix shown on the monitor 61, and may capture such images thereof for storage on the computer 60 or other storage media.

In the event that the examination device 50 is required to introduce an implement into the vagina, such as the sampling implement 15 as depicted in Fig. 1, an advantage of the above arrangement is that the physician may easily hold the handpiece 2 in one hand whilst manipulating the rear end of the implement 15 in the other hand.

The arrangement of the implement 15 extending through the lateral entry port 11 and tubular guide member 14 may at least enable the implement 15 to be far more easily manipulated than the arrangement of the earlier mentioned prior art. The present arrangement may at least allow for sampling devices or surgical instruments to be passed through the examination device 50 and into the vagina for taking samples or conducting minor surgery more easily than the prior art devices. An advantage of such an arrangement is that the physician can manipulate such sampling or surgical devices laterally of the examination device 50 and his/her hands do not block their field of vision, as is sometimes the case when using known colposcopes. Also, as the guide member 16 is offset from the longitudinal (i.e. central) axis of the tubular probe 1, the guide member 16 also does not have a large impact on the physician's field of vision.

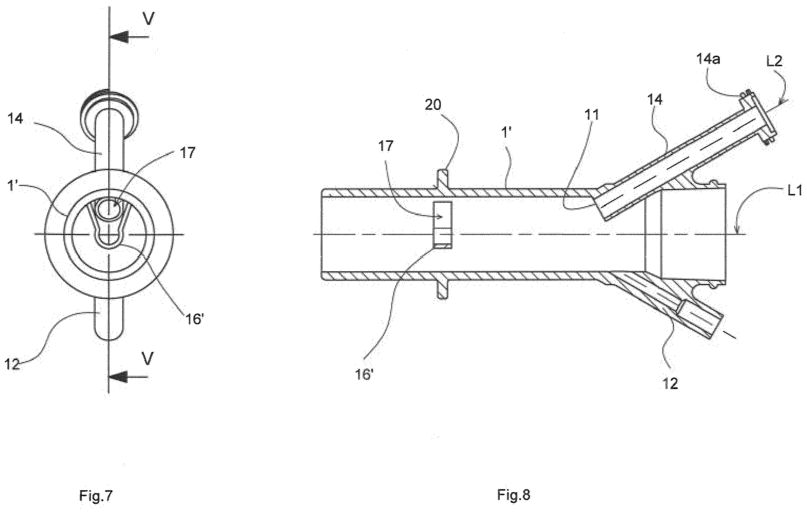

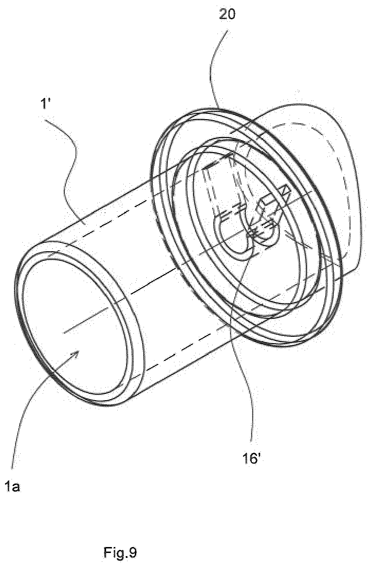

Figs. 7 to 9 show a variation of the tubular probe 1', which has a similar construction to the tubular probe 1 described above, with like reference numerals being used to indicate like features. However, in this variation, the tubular probe 1' includes a substantially keyhole-shaped guide member 16' projecting from the inner surface or walls of the bore 1a of the tubular probe 1.

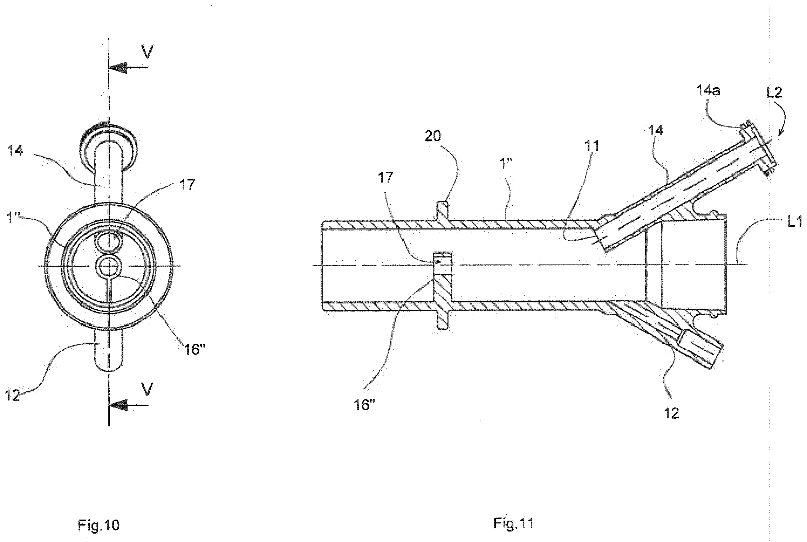

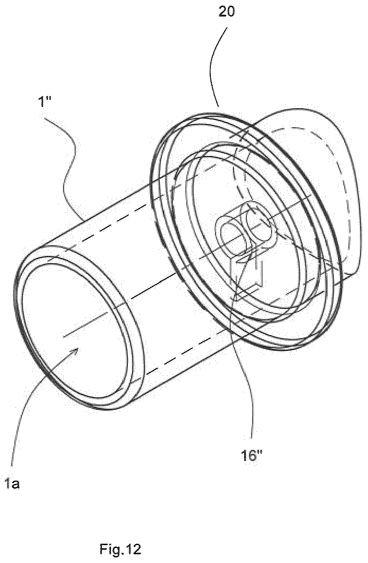

Figs. 10 to 12 show another variation of the tubular probe 1", which again has a similar construction to the tubular probe 1 described above, with like reference numerals being used to indicate like features. In this variation, the tubular probe 1" includes a substantially ring-shaped guide member 16" projecting from the inner surface or walls of the bore 1a of the tubular probe 1.

It is envisaged that the substantially keyhole-shaped guide member 16' and the substantially ring-shaped guide member 16" may at least allow for the implement 15 to be "dead-centered" within the tubular probe 1, such that the removal of the implement 15 from the vagina may occur without any issues. For example, when an implement such as a spatula is utilised, this arrangement at least allows the spatula to be centralised such that when the spatula is removed from the tubular probe 1 (i.e. pulled back into the tubular probe 1), the head of the spatula does not get left behind in the vagina, as is sometimes the case with prior art devices.

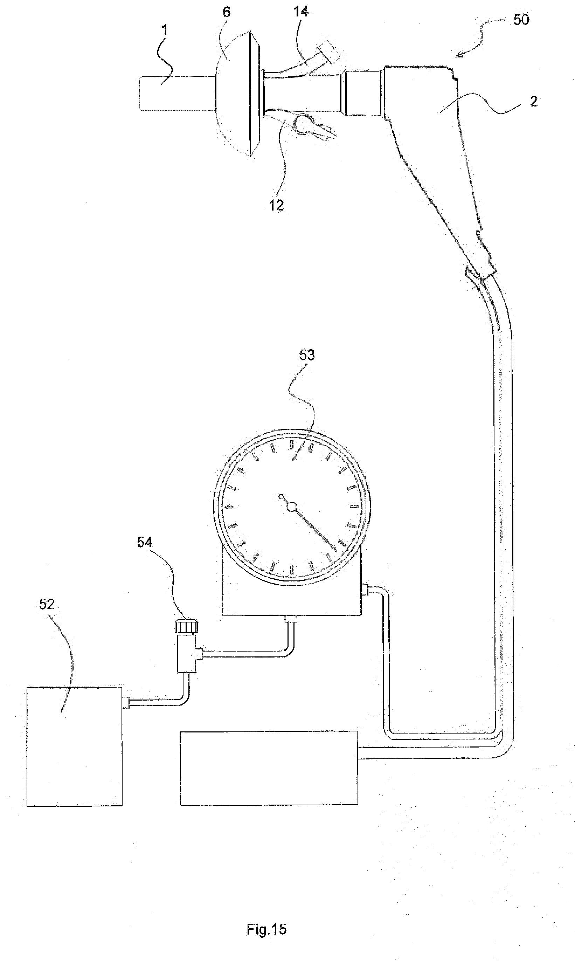

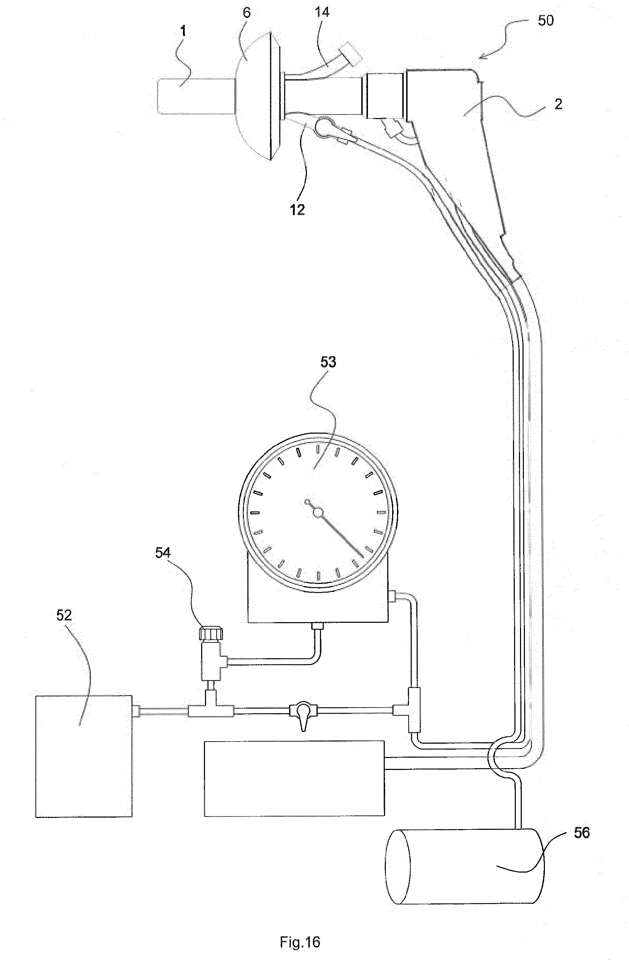

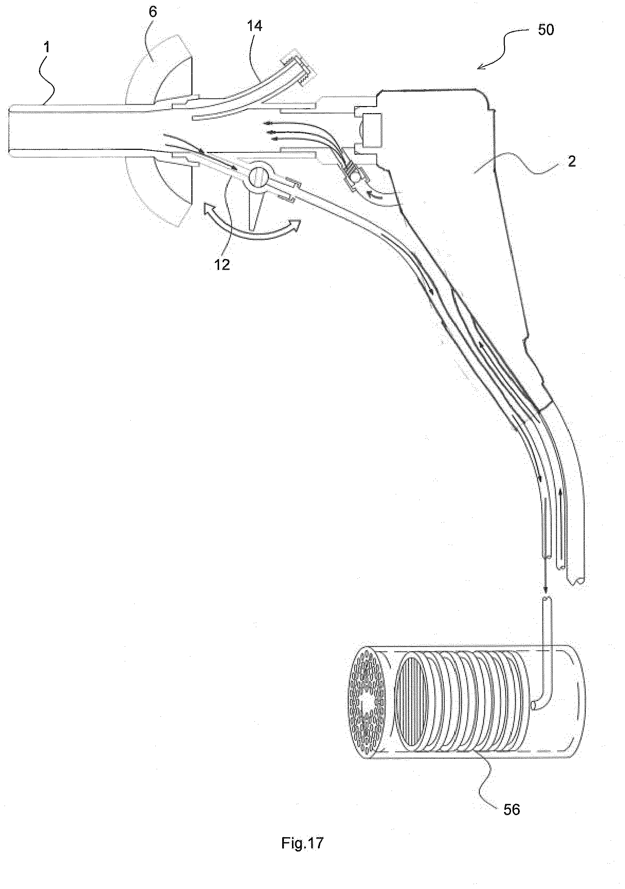

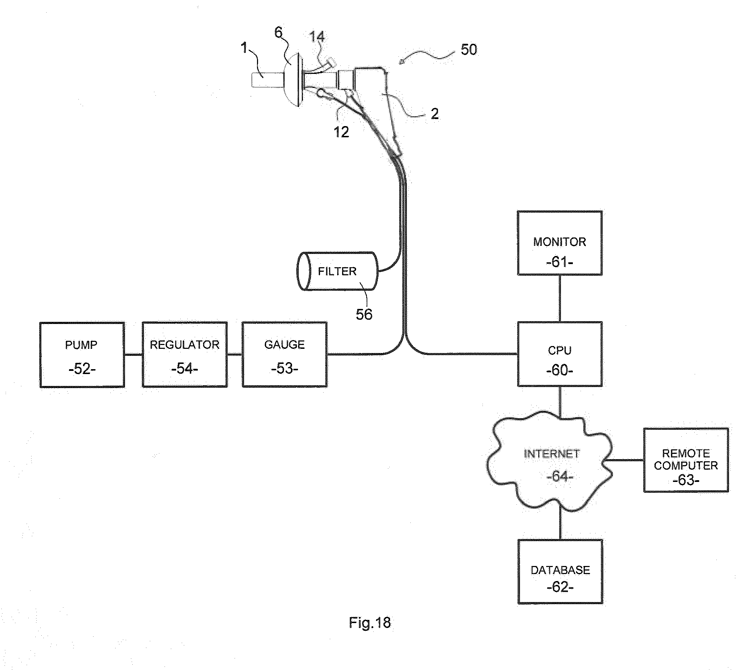

As best shown in Figs. 13 to 16, the examination device (speculum) 50 is fluidly connected via a conduit 51 to an air pump 52. Connected to the conduit 51 between the air pump 52 and the speculum 50 is a pressure gauge 53 and a pressure regulator 54. In another embodiment of the system comprising the examination device 50 as shown in Figs. 16 to 18, an air filter 56 may be connected to the fluid entry port 12, in conjunction with the conduit 51 or via a separate line or hose (not shown). The air filter 56 is operable by way of an on-off switch, which when turned on, would direct the air through the air filter 56. In one example, the air filter 56 would be located underneath a trolley, and behind the air pump 52. The air filter 56 may thus allow for the elimination of unwanted odours, which may typically emanate from the patient's vagina during the inflation procedure and escape into the room atmosphere. As such, issues with the air pressure and odour build-up in the patient's vagina may be avoided. This may also be of particular advantage when the same room is used to examine multiple patients, one after another, as the buildup and combination of unwanted odours in the room atmosphere may be avoided. As best seen in Figures 13 to 18, the air flow tubes travel along the side of the handpiece 2. The electrical wires travel through the back of the handpiece. Both tubes and wires end up into conduit 51.

The pressure gauge 53 may be either digital or analog, and adapted to display air pressure in a suitable pressure unit, such as mmHG. The pressure gauge 53 may, for example, be similar to those used in medical devices such as in blood pressure monitors, and may be integrally housed with the pressure regulator 54. In some circumstances, there may be a loss of air pressure due to air passing through the pressure gauge 53. To overcome this problem, a pressure gauge bypass tube or line (not shown) may be introduced, which is manually operable by the physician to turn to pressure gauge on or off.

The pressure regulator 54 allows for the adjustment of air pressure between at least a first, "upper limit" and a second, "lower limit". It is envisaged that the pressure range that the pressure regulator 54 operates within, is a range required to keep a typical vaginal barrel inflated when the sealing element 6 of the examination device (speculum) 50 is held sealingly against the PC muscle of a patient, i.e. the upper limit is below a pressure which causes air to escape from the sealing element 6, and the lower limit is above a minimal pressure to sufficiently inflate the typical vaginal barrel so that the cervix and surrounding area can be viewed by the camera.

The pressure regulator 54 may be a manual "turn knob" regulator, whereby the turn of the knob allows the adjustment of the pressure between the desired upper limit and the lower limit. In such an arrangement, the physician would insert the tubular probe 1 and abut the sealing element 6 against the vaginal vulva with sufficient force to affect the seal. Air would be then be introduced into the vagina from the pump 52, and with the physician looking at the pressure gauge 53, adjust the air pressure to a suitable level between the upper limit, i.e. just below where typical over-pressuring occurs that leads to air escaping the seal zone, and the lower limit which is just above the pressure needed to keep the vaginal barrel inflated.

In a series of air pressure trials at room temperature, it was shown that the air pressure does not go over 46 mmHg (about 6.13 kPa). As such, the range of suitable pressure is relatively low. It is therefore important to finely regulate the air between the upper and lower limits of the suitable pressure range.

In an alternative configuration, the pressure regulator 54 may be automated via the use of a pressure sensor (not shown) and an electronic control unit (not shown) integral with the pressure regulator 54. Such an automated pressure regulator 54 can automatically adjust the pressure so that it remains regulated between the upper limit and lower limit. It is envisaged that such an automated version of the pressure regulator 54 may be powered by the same power source as the air pump 52. It may also be possible, via the electronic control unit, to select between two or three predetermined pressure ranges, each with its own upper and lower limit, to allow the user different options to select from. This may at least allow for the adjustment of the actual pressure range, with slightly different ranges to accommodate patients with different vaginal barrel size and shape. Such an automated pressure regulator 54 may also have an alarm that is either a visual alarm or an audible alarm, operably connected to the electronic control unit, which sounds (e.g. beeps) or lights up, when the air pressure is sensed as being outside the predetermined range.

It is envisaged that the simplified regulation of air by the pressure regulator 54 described above may at least significantly improve a patient examination procedure in at least the following ways.

Firstly, the arrangement described above may at least minimise the risk of embarrassing patients by the noise caused by over-pressurised air escaping the sealing element 6 of the examination device (speculum) 50. Secondly, by simplifying and/or automating air pressure regulation, the user of the examination device (speculum) 50 has an easier task of conducting the patient examination procedure. By allowing for the patient examination procedure to be carried out in an easier and quicker manner, the patient examination procedure does not necessarily need to be carried out by a highly trained physician. The examination device 50 may thus be more cost effectively integrated into a "telehealth system" or the like.

In one example, by using the digital camera (not shown) in the handpiece 2 of the examination device 50 connected to the computer 60, in combination with the above-mentioned air pressure regulation by the pressure regulator 54, the patient examination procedure does not need to be carried out in the immediate presence of a physician. For example, a health professional other than a physician, such as a nurse, could readily operate the examination device 50. The simplified air pressure regulation via the pressure regulator 54 allows the nurse to concentrate on the image being shown on the monitor 61 via the computer (i.e. the central processing unit) 60. In one example, once the monitor 61 shows an image of the inflated vagina (with the cervix being shown at the centre of the monitor 61), the physician or nurse may turn down the pressure by observing the pressure gauge 53 and adjusting the pressure regulator 54 accordingly. The pressure may be maintained at any desired level by the physician or nurse. This may at least eliminate issues for the patient, such as embarrassing noise problems as a result of air escaping past the seal zone.

As the computer 60 may be connected to other computers in a local network, or to a remote database 62 and remote computer 63 via the internet 64 (as best shown schematically in Fig. 13), or by mobile applications, Wi-Fi, Bluetooth or the like, a physician does not need to be in the room at the time of the patient examination procedure. Should the nurse identify a problem by viewing the image on monitor 61, a physician in another remote location, for example, at the remote computer 63 (or mobile phone, tablet or the like), could be contacted and consulted for troubleshooting in real time as the patient examination procedure takes place. This may be particularly useful for rural health professionals, for example. A home examination kit is also envisaged where the patient examines themselves and emails or uploads the images to the health professional for confirmation of diagnosis.

It is also envisaged that the images captured may not be limited to being stored on the computer 60 or an associated storage device, but the images can additionally or alternatively be stored in the remote database 62 at a remote location. It is envisaged that the images may also be stored on a portable storage device such as a USB, hard drive or the like. As such, the physician or other health professional may examine the images at a later time, after the patient examination procedure had taken place. This may be used, for example as a tool for comparison and analysis from a patient's previous visit. It is also envisaged that a database of images may be collected over time, and with the use of an appropriate software, this may enable to identification of pre cancers (and potentially full cancers), in a similar manner to face recognition capabilities. Based on this database of images, it may be possible for physicians to identify problems with future patients in real time. In relation to court proceedings for rape victims, for example, the capture of images (from a relatively gentle procedure) may also allow for the collection of evidence to be presented in court. Another possible use of such a database would be for medical training purposes. For example, a medical professor may utilise the database of images to formulate tutorials and walk-throughs, whereby the images may be presented together with a voice-over explanation from the professor. As such, students may no longer be required to visit numerous patients in a hospital for training purposes, where in typical circumstances, only about 20 students may have the opportunity to look through a tool such as a bi-valve speculum for a limited number of examples of clinical situations available on the day. Using the database of images, it may be possible for an unlimited number of students to study a possible 50,000 (or more) examples available to them on any given day.

In the prior art, the disposable sealing element 6 has typically been manufactured from polyethylene foam, as it is relatively effective for sealing purposes and inexpensive. By utilising more effective air regulation via the pressure regulator 54 as described above, it may be possible to make the sealing element 6 from an even more cost-effective material, such as soft polystyrene foam or the like, and still provide an effective seal.

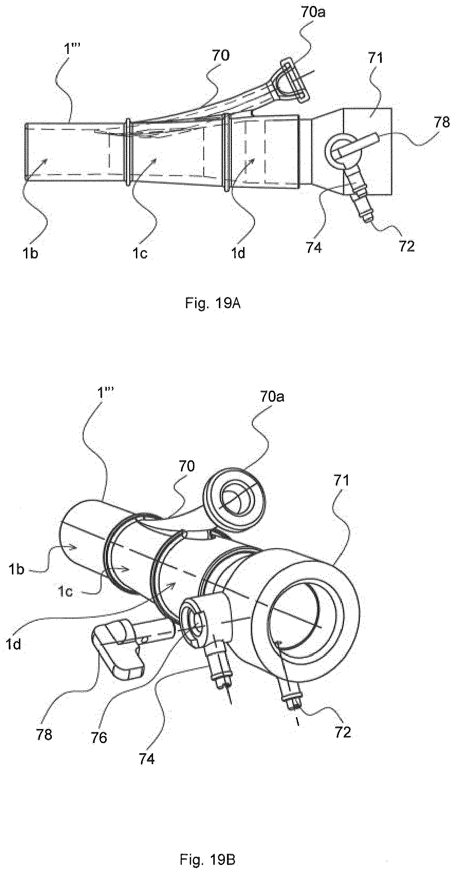

Figs. 19A and 19B show a variation of the tubular probe 1 of the examination device 50, being a gynecologist's speculum. In this embodiment, a tubular probe extension 1'" may replace the tubular probe 1 of the examination device shown in Fig. 1. The tubular probe extension 1'" includes access port 70 extending from the body of the tubular probe extension 1'". It is envisaged that a disposable portion 70a may be removably attachable to the access port 70. It will be appreciated that the tubular probe extension 1'", the access port 70 and the disposable portion 70a are each adapted to be removable and disposable, such that any implement or instrument (e.g. implement 15) that comes in contact with the patient's bodily fluids or tissue does not contaminate the rest of the examination device 50. For example, the access port 70 may provide a point of access for the physician to insert a tube through which a fluid such as acetic acid, for example, may be introduced into the vaginal barrel and on to the surface of the cervix to allow for an observation of white spots, which is an indication of precancerous cells. Once the procedure is complete, the physician simply has to remove and dispose of the tubular probe extension 1"', the access port 70 and/or the disposable portion 70a. The tubular probe extension 1"', the access port 70 and the disposable portion 70a may be formed as a single, integrally formed piece, or alternatively be formed from a number of interconnecting pieces, so as to accommodate different types of patient examination procedures and hygiene requirements. It should also be appreciated that the access port 70 may be adapted for the insertion of an intrauterine device (IUD) or the like to facilitate installation of the IUD (or the like) in a patient's uterus via the vagina.

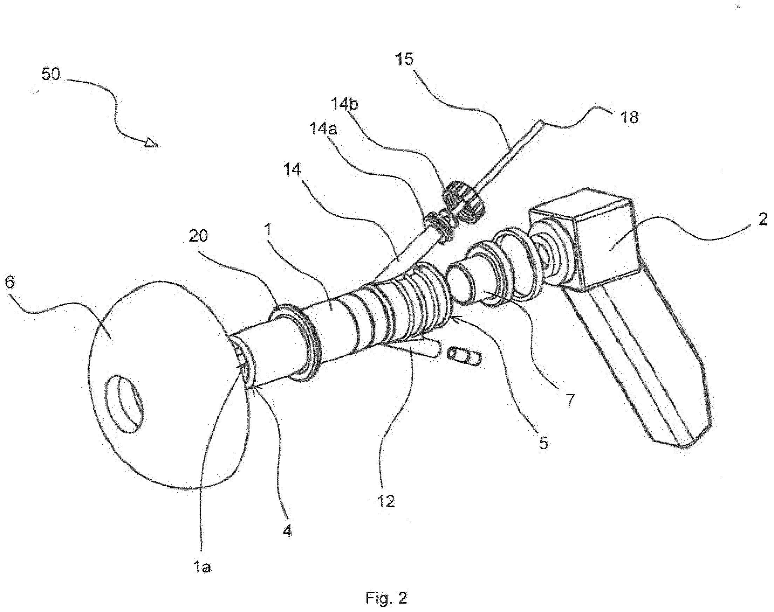

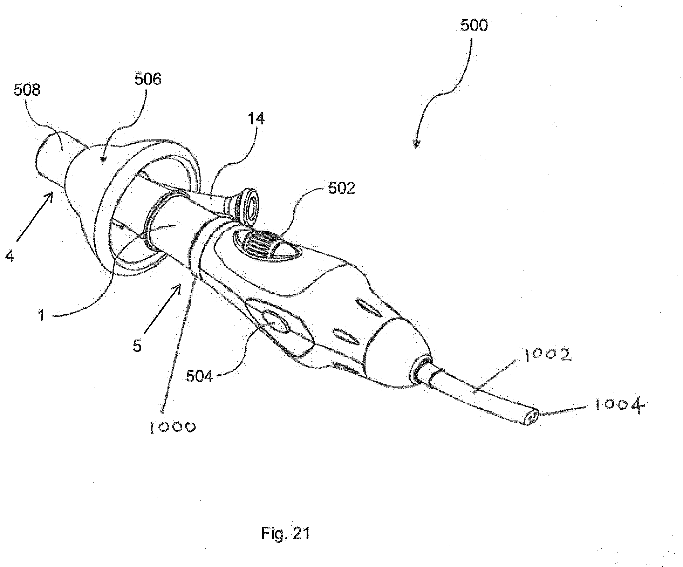

It is further envisaged that the gynecologist's speculum may include a port component 71 attached to the tubular probe extension 1"'. The port component 71 includes an air entry port 72, which extends from the body of the port component 71 to allow for the introduction of air or the like into the vaginal barrel. An air exit port 74 also extends from the port component 71 to allow for contaminated air to exit the tubular probe extension 1"', and subsequently be directed to the air filter 56 located behind the air pump 52. An air outlet 76 and associated tap member 78 extending from the port component 71 is also provided as the outlets from the tubular probe extension 1'". It should be appreciated that in one arrangement, the port component 71 may be adapted to replace the mounting spigot 7 of the examination device as shown in Fig. 2. That is component 71 would attach to part 1000 in Figure 21. This also has the benefit of saving internal use of the airway paths.

In the embodiment shown in Figs. 19A and 19B, the tubular probe extension 1'" may be formed as an integral component, or alternatively multiple components such as different tubular probe extension components 1b, 1c and Id, the access port 70, and the disposable portion 70a, which may be joined together by way of a press-fit or a thread-fit or the like.

In an alternative embodiment, the arrangement shown in Fig. 19A and 19B may be customised to have a longer and/or narrower body and utilised with a smaller sealing element 6. As such, the arrangement may be used as an anal pathology implement. The use of the pressure gauge 53 and the pressure regulator 54 may be beneficial in making certain anal examination procedures easier, as it allows for the practitioner to adjust the level of air going into a patient's anus and thus provide easier access. In the arrangements described above, the various components may be formed as disposable components to avoid contamination.

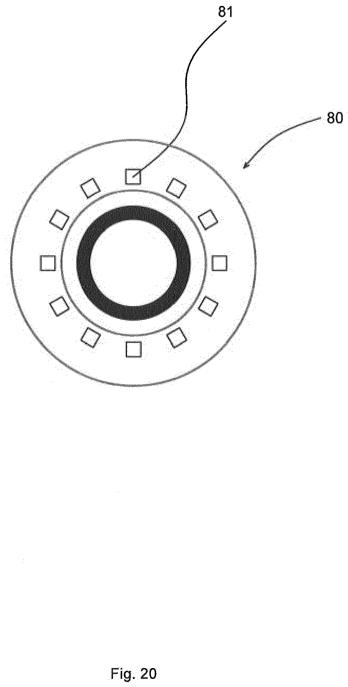

With reference to Fig. 20, an illumination assembly in the form of a light-emitting diode (LED) light array 80 is shown. The illumination assembly may be operably associated with the handpiece 2 and attached to the first (forward) end 4 of the tubular probe 1. This allows for the vaginal barrel to be lit up for the physician to view, and for images to be captured more clearly via the camera or other visual recording device. It is envisaged that this LED light array arrangement 80 includes a plurality of LEDs 81 that may alternate between white, green, red or UV LEDs. A dial (not shown) is operably associated with the LED light array arrangement 70 to allow a user to switch between different LED colours. In order to have richer colours to diagnose the images captured by the camera, it is further envisaged that the monitor 61 associated with the examination device 50 is provided with an organic light-emitting diode (OLED) screen.

Fig. 21 depicts a further embodiment of an examination device 500 intended for use in the examination of a vagina or other body cavity. The examination device 500 functions in a similar manner to the examination device 50 described above, with like reference numerals being used to depict like features. However, in this embodiment, the examination device 500 is a portable examination device having a smaller, more streamlined arrangement than the examination device 50 described above. The examination device 500 is battery-operable (i.e. not physically connected to a mains power source) to allow for ease of transport and manipulation. It is envisaged that the examination device 500 may be recharged by connecting to a 5V power source such as via a laptop or tablet computer (e.g. in the same way that a mobile phone device may be recharged). It is also envisaged that the examination device 500 may be include solar charging capabilities, which may at least allow the examination device 500 to be operational and rechargeable in remote areas where mains power may not be readily accessible.

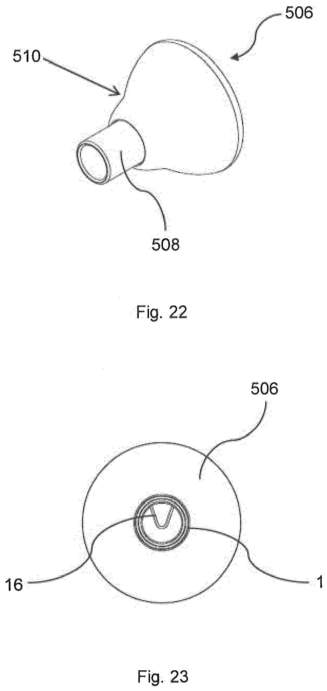

In this embodiment, the examination device 500 includes actuation member 502 (zoom in and out) on the top of the device and 504 on both sides of the device for taking images and video, which are preferably in the form of dials or buttons, but may be any other form of actuation mechanism. The actuation members 502 and 504 are operable by the user to control the examination device 500. The examination device 500 also includes a sealing element 506 (cup) disposed adjacent to the forward end 4 of the tubular probe 1, whereby the sealing element 506 includes a self-tightening sleeve portion 508 to engage the tubular probe 1. It will be appreciated that the sleeve portion 508 is biased to a diameter that is smaller than the diameter of the tubular probe 1, and the sleeve portion 508 may be stretched to fit over the tubular probe 1. Upon engagement with the tubular probe 1, the sleeve portion 508 tightens over the tubular probe 1 to secure it in place relative to the tubular probe 1. The device 500 may also have a conduit 1002 which houses the air out, air in tubes and the camera, light and power cables 1004. In the preferred embodiment as shown in Fig. 22, the sealing element 506 has a contoured outer profile with a gradually increasing diameter from a front end to a rear end, and with a slight step or indentation 510 in between. This contoured outer profile is designed to create an optimum fit with the inner walls of the vagina so as to provide a robust seal therebetween when the probe 1 is inserted into the vagina. This contoured outer profile may at least prevent air from escaping from the vagina during inflation and/or subsequent examination. It will be appreciated that the sealing element 506 may be provided in a range of other shapes and sizes to suit the patient's needs. For example, the sealing element 506 and tubular probe 1 may be provided in a variety of sizes identified by size A (for small children and older women who have smaller vaginas after menopause), size B (for the majority of women), or size C (for obese or larger women). The sealing element 506 is preferably formed of soft polyurethane material or the like. In other embodiments it could be autoclave hard plastic for reuse.

In Fig. 23, a further variation of the tubular probe 1 is depicted, and in this variation, the tubular probe 1 includes a substantially V-shaped guide member 16 projecting from the inner surface or walls of the bore 1a of the tubular probe 1. This substantially V-shaped guide member may at least allow for the implement 15 to be "dead-centered" within the tubular probe 1, and be supported in position so that the implement 15 does not move around. Again, as described above, this arrangement may at least prevent the risk of an implement, e.g. a spatula, from being caught on the end of the tubular probe 1. That is, when an implement such as a spatula is utilised, this arrangement at least allows the spatula to be centralised such that when the spatula is removed from the tubular probe 1 (i.e. pulled back into the tubular probe 1), the head of the spatula does not get left behind in the vagina, as is sometimes the case with prior art devices.

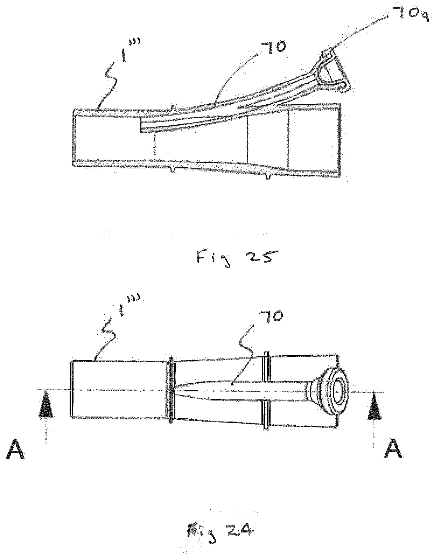

Further in Figures 24 and 25 a further tubular probe is shown. A flexible biopsy tool (not shown) can be guided through to stay in the top section and can be seen on the screen when a biopsy specimen is needed.

It is envisaged that the examination device 500 may be associated with an odour evacuation system. In embodiments, the odour evacuation system includes the air filter 56 described above to evacuate or eliminate unwanted odours from the patient's vagina. The odour evacuation system and the air filter 56 is operable by the practitioner using an on-off switch that is disposed either directly on the examination device 500, or remote from the examination device 500.

The examination device 500 may also further be associated with the pressure gauge 53 and the pressure regulator 54 described above, which are connected to the conduit 51 and the air pump 52. The pressure gauge 53 is operable by the practitioner to set the air pressure, e.g. in the predetermined range of pressure as required. The pressure regulator 54 is operable to regulate the air pressure inside the patient's vagina. In embodiments, the examination device may be further associated with one or more pressure valves that allow the practitioner to select a predetermined pressure according to the size of the patient's vagina. For example, the practitioner may select a predetermined pressure "A" which corresponds to size A of the sealing element 506. This may at least provide ease of usability and convenience for the practitioner, as they would no longer need to manually set the pressure for each patient. It will be appreciated that the selection of the predetermined pressure using the one or more pressure valves by the practitioner overrides any existing pressure that was previously set.

In embodiments, it is envisaged that the examination device 500 may further include an imaging device having an associated light source for emitting green, white, or ultraviolet blue light onto the vaginal walls or cervix. The imaging device may be a standard (2D) camera or camera with 3D imaging capabilities. It will be appreciated that the emission of green or blue light on to the vaginal walls or cervix may enable the identification of cancerous spots on the vaginal walls after acetic acid (mild vinegar) is sprayed onto the vagina (e.g. via the port 12 or 14). Following the application of acetic acid, cancerous spots typically show up as white spots on the vaginal walls or cervix. The green or blue light assist with making these white spots stand out for better identification.

It is envisaged that the identification of cancerous spots on the vaginal walls or cervix may be enhanced by incorporating machine learning capabilities, such that the identification from the image capture of the cancerous spots may be automated. Such automation may also allow tiny pixels of cancerous spots from as littles as 18 pixels of white (which are not visible to the human eye) to be picked up by the associated software, thus improving the efficiency and accuracy of identifying the cancerous spots. By incorporating such automation capabilities, the identification of cancerous spots may be performed without involving pathology, which is highly beneficial in remote areas where a pathology lab may not be available for hundreds of kilometers. The automatic detection of the cancerous spots may at least allow a nurse (instead of a trained gynecologist) to operate the examination device 500 and confirm the presence of cancerous spots, without necessarily having to submit samples for testing or having a trained gynecologist inspect the captured images. It is envisaged that the image capture may be overlaid with a grid to assist the practitioner with locating the cancerous spots.

The image capture may also be localised, or alternatively transmitted as real-time images to a practitioner in a different location. It is also envisaged that the image capture may be associated with Virtual Reality (VR) technology to allow for more accurate viewing and analysis. For example, the imaging device may be connectable to a VR headset or screen to allow the practitioner to have a closer and more realistic view of the cervix and/or vaginal walls, which would assist with locating and identification of any potential issues (e.g. cancerous spots or other anomalies).

It will be appreciated that the automated detection of cancerous spots may be enhanced by the collection of data of images that have confirmed presence of cancer on the cervix or vaginal walls, or even STDs. The collection and compilation of images may allow for the machine to continually learn the patterns associated with the confirmed cancerous spots and increase its identification accuracy.

It will also be appreciated that the incorporation of the imaging device into the examination device may also be utilised to examine the vagina to assist with forensic rape cases and to produce evidence of assault or physical trauma. The imaging device may also be used to produce photographic or videographic case studies for educational purposes, e.g. for medical students to study and learn how to treat the various types of cancers, STDs and to identify and locate foreign objects inside the vagina.

It is envisaged that the portability and ease of operation of the examination device described above may allow a patient to purchase and use the device in their own home, and to send the results to their practitioner for evaluation. As such, both the patient and practitioner can save time and costs associated with the initial screening process. The practitioner may then be able to determine if it is necessary for that patient to travel to the clinic for treatment.

As embarrassment is a major issue in the above patient examination procedures, it is envisaged that robotic arms or the like may be introduced in conjunction with the examination device and systems described above. This would ideally be designed such that the robotic arms may be operated remotely by the physician (so that they are not required to be in the same room as the patient). The robotic arms would ideally have a human touch and operate "hand-in-glove". This may at least eliminate the embarrassment obstacle, for example with patients who undergo cervical cancer tests and procedures.

It is further envisaged that the examination device and system described above may also be adapted to facilitate x-rays or other scanning methods. The examination device and system may also be made in a compact form so as to be portable, e.g. transported around in a suitcase or the like, such that the device and system may be mobile and implemented in remote areas.

It is also envisaged that the examination device and system described above may be custom-fit, depending on the type of examination procedure taking place, and also the type of patient being examined. For example, the tubular probe and sealing element described above may be adjusted such that they are smaller in diameter and overall size for women with smaller vaginal cavities, or for examining patients of a younger age. With reference to Fig. 1, for example, the length x between the first forward end 4 of the tubular probe 1 and the sealing element 6 may be designed to be about 3 to 4 cm in length to allow for sufficient access into a vaginal cavity prior to the introduction of air pressure. As such, excessive force is not required to insert the tubular probe into a patient. The examination device may then be pivoted around for a complete view of the vaginal cavity walls (i.e. the interior of the vagina), a functionality that is not possible in prior art devices. The examination device and system described above are also designed with ease of disposal in mind, with the various components such as the tubular probe and sealing element (when being used as a doctor's speculum), and the tubular probe extension, access port and disposable portion (when being used as a gynecologist's speculum) being disposable for hygiene purposes.

Although the invention has been described with reference to preferred embodiments, it will be appreciated by persons skilled in the art that the invention may be embodied in many other forms.

## Claims

1. A portable device for examining a body cavity, the device adapted to allow air to be introduced into the body cavity, the device comprising:
a tubular probe having a first, forward end and a second, rearward end; and
a sealing element disposed adjacent to the forward end of the tubular probe, the sealing element including a self-tightening sleeve portion to engage the tubular probe, the sealing element adapted to act as a seal for the body cavity to reduce egress of fluid therefrom by sealing against a circumferential zone of the internal tissue defining an orifice of the body cavity,
the tubular probe having a first lateral entry port that allows for an elongate implement to be removably mounted in the device and to be passed through the tubular probe for introduction into the body cavity, such that the implement is manipulable by a user,
the portable device being connectable to an odour evacuation system having a filter to remove the build-up of air in the body cavity, and
wherein fluid being introduced into said body cavity via the tubular probe is delivered via a conduit from a pump, and a regulator fluidly connected to the conduit regulates the fluid to be within at least one predetermined range of pressure between at least an upper limit and a lower limit.

2. A device as claimed in claim 1, wherein a pressure gauge is in fluid communication with the conduit for setting the predetermined range of pressures.

3. A device as claimed in claim 1 or 2, wherein the upper limit is just below where over-pressuring of air occurs in the vagina, and the lower limit is just above a pressure needed to keep the body cavity inflated for viewing.

4. A portable device as claimed in any one of claims 1 to 3, wherein the tubular probe includes a guide means disposed adjacent the second, rearward end of the prove, the guide means adapted to guide the implement within the tubular probe to stay adjacent a top section of the probe, and wherein the guide means is a substantially V-shaped guide member.

5. A portable device as claimed in any one of claims 1 to 4, further including an imaging device having an associated light source for emitting green, white, or ultraviolet blue light onto the body cavity.
